# FASCICULE DE BREVET EUROPEEN

(11) **EP 0 703 980 B1**
(45) Date de publication et mention de la délivrance du brevet: **29.01.2003**
(21) Numéro de dépôt: 94919711.5
(22) Date de dépôt: 15.06.1994
(51) Int. Cl.: C12N 15/12, C12N 15/13

(54) **PROTEINES INTRACELLULAIRES DE LIAISON (PIL) ET LEURS UTILISATIONS**
INTRAZELLULAERE BINDUNGS PROTEINE UND DEREN ANWENDUNGEN
INTRACELLULAR BINDING PROTEINS AND USE THEREOF

(30) Priorité: 16.06.1993 FR 9307241
(43) Date de publication de la demande: 03.04.1996
(73) Titulaire: Aventis Pharma S.A., 92160 Antony (FR)
(72) Inventeur: SCHWEIGHOFFER, Fabien, F-94300 Vincennes (FR); TOCQUE, Bruno, F-75017 Paris (FR)
(74) Mandataire: Becker, Philippe
(86) Numéro de dépôt international: FR9400714
(87) Numéro de publication internationale: WO94029446

(56) Documents cités:
- DE-A- 3 915 952
- NATURE., vol.342, 2 Novembre 1989, LONDON GB pages 99 - 100 RODWELL, J.D. 'Engineering monoclonal antibodies'
- NUCLEIC ACIDS RESEARCH., vol.21, no.5, 11 Mars 1993, ARLINGTON, VIRGINIA US pages 1081 - 1085 RUSSELL, S.J. ET AL.; 'Retroviral vectors displaying functional antibody fragments.'

### LISTE DE SEQUENCES

### (1) INFORMATION GENERALE:

(i) DEPOSANT:
   (A) NOM: RHONE-POULENC RORER S.A.
   (B) RUE: 20, avenue R. ARON
   (C) VILLE: ANTONY
   (E) PAYS: FRANCE
   (F) CODE POSTAL: 920165
(ii) TITRE DE L' INVENTION: Séquences nucléiques, vecteurs les contenant, compositions pharmaceutiques et utilisations thérapeutiques.
(iii) NOMBRE DE SEQUENCES: 10
(iv) FORME LISIBLE PAR ORDINATEUR:
   (A) TYPE DE SUPPORT: Tape
   (B) ORDINATEUR: IBM PC compatible
   (C) SYSTEME D' EXPLOITATION: PC-DOS/MS-DOS
   (D) LOGICIEL: PatentIn Release #1.0, Version #1.25 (OEB)

### (2) INFORMATION POUR LA SEQ ID NO: 1:

(i) CARACTERISTIQUES DE LA SEQUENCE:
   (A) LONGUEUR: 870 paires de bases
   (B) TYPE: acide nucléique
   (C) NOMBRE DE BRINS: double
   (D) CONFIGURATION: linéaire
(ii) TYPE DE MOLECULE: ADNc
(iii) HYPOTHETIQUE: NON
(iii) ANTI-SENS: NON
(ix) CARACTERISTIQUE ADDITIONELLE:
   (A) NOM/CLE: CDS
   (B) EMPLACEMENT: 1..870
(ix) CARACTERISTIQUE ADDITIONELLE:
   (A) NOM/CLE: misc_feature
   (B) EMPLACEMENT: 442..486
   (D) AUTRES RENSEIGNEMENTS: /product= "Linker"
(ix) CARACTERISTIQUE ADDITIONELLE:
   (A) NOM/CLE: misc_feature
   (B) EMPLACEMENT: 82..810
   (D) AUTRES RENSEIGNEMENTS: /product= "ScFv anti-Ras"
(xi) DESCRIPTION DE LA SEQUENCE: SEQ ID NO: 1:
   TCA AGG AGA CAG TCT ATA AGA AAT ACC TAT TCG ACG GCA GCC GCT GGA      48
   Ser Arg Arg Gln Ser Ile Arg Asn Thr Tyr Ser Thr Ala Ala Ala Gly
     1               5                  10                  15
   TTG TTA TTA CTC GCG GCC CAG CCG GCC ATG GCT CAG GTG AAA CTG CAG      96
   Leu Leu Leu Leu Ala Ala Gln Pro Ala Met Ala Gln Val Lys Leu Gln
                20                  25                  30
   CAG TCA GGA GGA GGC TTA GTG CAG CCT GGA AGG TCC CTG AAA CTC TCC     144
   Gln Ser Gly Gly Gly Leu Val Gln Pro Gly Arg Ser Leu Lys Leu Ser
            35                  40                  45
   TGT GTA GTC TCT GGA TTC ACT TTC AGT AAC TAT GGA ATG AAC TGG ATT     192
   Cys Val Val Ser Gly Phe Thr Phe Ser Asn Tyr Gly Met Asn Trp Ile
        50                  55                  60
   CGC CAG ACT CCA GGG AAG GGA CTG GAG TGG GTT GCA TAC ATT AGT AGT     240
   Arg Gln Thr Pro Gly Lys Gly Leu Glu Trp Val Ala Tyr Ile Ser Ser
    65                  70                  75                  80
   GGT AGC AGT TAC CTC TAC TAT GCA GAA ACG GTG AAG GGC CGA TTC ACC     288
   Gly Ser Ser Tyr Leu Tyr Tyr Ala Glu Thr Val Lys Gly Arg Phe Thr
                    85                  90                  95
   ATC TCC AGA GAC AAT GCC AAG AAC ACC CTG TAC CTG CAA ATG ACC AGT     336
   Ile Ser Arg Asp Asn Ala Lys Asn Thr Leu Tyr Leu Gln Met Thr Ser
               100                 105                 110
   CTG AGG TCT GAA GAC ACT GCC TTG TAT TAC TGT GCA AGA CAT GAG GGT     384
   Leu Arg Ser Glu Asp Thr Ala Leu Tyr Tyr Cys Ala Arg His Glu Gly
           115                 120                 125
   ACG GGT ACC GAC TTC TTT GAT TAC TGG GGC CAA GGG ACC ACG GTC ACC     432
   Thr Gly Thr Asp Phe Phe Asp Tyr Trp Gly Gln Gly Thr Thr Val Thr
       130                 135                 140
   GTC TCC TCA GGT GGA GGC GGT TCA GGC GGA GGT GGC TCT GGC GGT GGC     480
   Val Ser Ser Gly Gly Gly Gly Ser Gly Gly Gly Gly Ser Gly Gly Gly
   145                 150                 155                 160
   GGA TCG GAC GTT GAG CTC ACC CAG TCT CCA CAT TCC CTG TCT GCA TCT     528
   Gly Ser Asp Val Glu Leu Thr Gln Ser Pro His Ser Leu Ser Ala Ser
                   165                 170                 175
   CTG GGA GAA ACT GTC TCC ATC GAA TGT CTA GCA AGT GAG GGC ATT TCC     576
   Leu Gly Glu Thr Val Ser Ile Glu Cys Leu Ala Ser Glu Gly Ile Ser
               180                 185                 190
   AAT TAT TTA GCG TGG TAT CAG CAG AAG CCA GGG AAA TCT CCT CAG CTC     624
   Asn Tyr Leu Ala Trp Tyr Gln Gln Lys Pro Gly Lys Ser Pro Gln Leu
           195                 200                 205
   CTG ATC TAT TAT GCA AGT AGC TTG CAG GAT GGG GTC CCA TCA CGG TTC     672
   Leu Ile Tyr Tyr Ala Ser Ser Leu Gln Asp Gly Val Pro Ser Arg Phe
       210                 215                 220
   AGT GGC AGT GGA TCT GGC ACA CAG TTT TCT CTC AAG ATC AGC AAC ATG     720
   Ser Gly Ser Gly Ser Gly Thr Gln Phe Ser Leu Lys Ile Ser Asn Met
   225                 230                 235                 240
   CAA CCT GAA GAT GAA GGG GTT TAT TAC TGT CAA CAG GCT TAC AAG TAT     768
   Gln Pro Glu Asp Glu Gly Val Tyr Tyr Cys Gln Gln Ala Tyr Lys Tyr
                   245                 250                 255
   CCT TCC ACG TTT GGA GCT GGC ACC AAG CTG GAA ATA AAA CGG GCG GCC     816
   Pro Ser Thr Phe Gly Ala Gly Thr Lys Leu Glu Ile Lys Arg Ala Ala
               260                 265                 270
   GCA GAA CAA AAA CTC ATC TCA GAA GAG GAT CTG AAT TAA TAA GAA TTC     864
   Ala Glu Gln Lys Leu Ile Ser Glu Glu Asp Leu Asn  *   *  Glu Phe
           275                 280                 285
   ACT GGC                                                        870
   Thr Gly
       290

### (2) INFORMATION POUR LA SEQ ID NO: 2:

(i) CARACTERISTIQUES DE LA SEQUENCE:
   (A) LONGUEUR: 810 paires de bases
   (B) TYPE: acide nucléique
   (C) NOMBRE DE BRINS: double
   (D) CONFIGURATION: linéaire
(ii) TYPE DE MOLECULE: ADNc
(iii) HYPOTHETIQUE: NON
(iii) ANTI-SENS: NON
(ix) CARACTERISTIQUE ADDITIONELLE:
   (A) NOM/CLE: CDS
   (B) EMPLACEMENT: 1..810
(ix) CARACTERISTIQUE ADDITIONELLE:
   (A) NOM/CLE: misc_feature
   (B) EMPLACEMENT: 382..426
   (D) AUTRES RENSEIGNEMENTS: /product= "Linker"
(ix) CARACTERISTIQUE ADDITIONELLE:
   (A) NOM/CLE: misc_feature
   (B) EMPLACEMENT: 31..753
   (D) AUTRES RENSEIGNEMENTS: /product= "ScFv anti-GAP"
(xi) DESCRIPTION DE LA SEQUENCE: SEQ ID NO: 2:
   TTA TTA CTC GCG GCC CAG CCG GCC ATG GCC CAG GTC CAA CTG CAG GAG     48
   Leu Leu Leu Ala Ala Gln Pro Ala Met Ala Gln Val Gln Leu Gln Glu
    1               5                  10                  15
   TCA GGA CCT GGC CTA GGG CAG CCC GCA CAG AGC ATT TCC ATA ACC TGC     96
   Ser Gly Pro Gly Leu Gly Gln Pro Ala Gln Ser Ile Ser Ile Thr Cys
                20                  25                  30
   ACA GTC TCT GGT TTC TCA TTA AGT AGC TAT GGT GTA CAC TGG GTT CGC    144
   Thr Val Ser Gly Phe Ser Leu Ser Ser Tyr Gly Val His Trp Val Arg
            35                  40                  45
   CAG TCT CCA GGA AAG GGT CTG GAG TGG CTG GGA GTG ATA TGG AGA GGT    192
   Gln Ser Pro Gly Lys Gly Leu Glu Trp Leu Gly Val Ile Trp Arg Gly
        50                  55                  60
   GGA GGC ACA GAC TAC AAT GCA GCC TTC ATG TCC AGA CTG AGC ATC ACC    240
   Gly Gly Thr Asp Tyr Asn Ala Ala Phe Met Ser Arg Leu Ser Ile Thr
    65                  70                  75                  80
   AAG GAC AAC TCC AAG AGC CAA GTT TTC TTT AAA TTG AAC AGT CTG CAA    288
   Lys Asp Asn Ser Lys Ser Gln Val Phe Phe Lys Leu Asn Ser Leu Gln
                    85                  90                  95
   CCT GAT GAC ACT GCC ATG TAC TAC TGT GCC AAA AGG GGT GGC CCG GGG    336
   Pro Asp Asp Thr Ala Met Tyr Tyr Cys Ala Lys Arg Gly Gly Pro Gly
               100                 105                 110
   TAT TTC GAT GTC TGG GGC CAA GGG ACC ACG GTC ACC GTC TCC TCA GGT     384
   Tyr Phe Asp Val Trp Gly Gln Gly Thr Thr Val Thr Val Ser Ser Gly
           115                 120                 125
   GGA GGC GGT TCA GGC GGA GGT GGC TCT AGC GGT GGC GGA TCG GAC ATT     432
   Gly Gly Gly Ser Gly Gly Gly Gly Ser Ser Gly Gly Gly Ser Asp Ile
       130                 135                 140
   GAG CTC ACC CAG TCT CCA GCC TCC CTA TCT GCA TCT GTG GGA GAA ACT     480
   Glu Leu Thr Gln Ser Pro Ala Ser Leu Ser Ala Ser Val Gly Glu Thr
   145                 150                 155                 160
   GTC ACC ATG ACA TGT CGA GCA AGT GAG AAT ATT TAC AGT AAT TTA GCA     528
   Val Thr Met Thr Cys Arg Ala Ser Glu Asn Ile Tyr Ser Asn Leu Ala
                   165                 170                 175
   TGG TAT CAG CAG AAA CAG GGA AAG TCT CCT CAG CTC CTG GTC TAT GCT     576
   Trp Tyr Gln Gln Lys Gln Gly Lys Ser Pro Gln Leu Leu Val Tyr Ala
               180                 185                 190
   GCA ACA AAA CCA GGA AAT GGT GTG CCA TCA AGG TTC AGT GGC AGT GGA     624
   Ala Thr Lys Pro Gly Asn Gly Val Pro Ser Arg Phe Ser Gly Ser Gly
           195                 200                 205
   TCA GGC ACA CAA TTT TCT CTG AAG ATC AAC AGC CTG CAG CCT GAA GAT     672
   Ser Gly Thr Gln Phe Ser Leu Lys Ile Asn Ser Leu Gln Pro Glu Asp
       210                 215                 220
   CTT GGG AAC TAT TAC TGT CTA CAT TTT TAT GGG ACT CCG TAT AGG TTC     720
   Leu Gly Asn Tyr Tyr Cys Leu His Phe Tyr Gly Thr Pro Tyr Arg Phe
   225                 230                 235                 240
   GGC GGG GGC ACC AAG CTG GAA ACG AAA CGG GCG GCC GCA GAA CAA AAA     768
   Gly Gly Gly Thr Lys Leu Glu Thr Lys Arg Ala Ala Ala Glu Gln Lys
                   245                 250                 255
   CTC ATC TCA GAA GAG GAT CTG AAT TAA TAA GAA TTC ACT GGC             810
   Leu Ile Ser Glu Glu Asp Leu Asn  *   *  Glu Phe Thr Gly
               260                 265                 270

### (2) INFORMATION POUR LA SEQ ID NO: 3:

(i) CARACTERISTIQUES DE LA SEQUENCE:
   (A) LONGUEUR: 11 acides aminés
   (B) TYPE: acide aminé
   (D) CONFIGURATION: linéaire
(xi) DESCRIPTION DE LA SEQUENCE: SEQ ID NO: 3:
   Pro Val Glu Asp Arg Arg Arg Val Arg Ala Ile
   1               5                   10

### (2) INFORMATION POUR LA SEQ ID NO: 4:

(i) CARACTERISTIQUES DE LA SEQUENCE:
   (A) LONGUEUR: 4 acides aminés
   (B) TYPE: acide aminé
   (D) CONFIGURATION: linéaire
(xi) DESCRIPTION DE LA SEQUENCE: SEQ ID NO: 4:
   Glu Ile Ser Phe
    1

### (2) INFORMATION POUR LA SEQ ID NO: 5

(i) CARACTERISTIQUES DE LA SEQUENCE:
   (A) LONGUEUR: 5 acides aminés
   (B) TYPE: acide aminé
   (D) CONFIGURATION: linéaire
(xi) DESCRIPTION DE LA SEQUENCE: SEQ ID NO: 5:
   Glu Asp Gly Trp Met
    1               5

### (2) INFORMATION POUR LA SEQ ID NO: 6

(i) CARACTERISTIQUES DE LA SEQUENCE:
   (A) LONGUEUR: 25 acides aminés
   (B) TYPE: acide aminé
   (D) CONFIGURATION: linéaire
(xi) DESCRIPTION DE LA SEQUENCE: SEQ ID NO: 6
   Gln Tyr Arg Leu Lys Lys Ile Ser Lys Glu Glu Lys Thr Pro Gly Cys Val
    1               5                  10                  15
   Lys Ile Lys Lys Cys Ile Ile Met
            20                  25

### (2) INFORMATION POUR LA SEQ ID NO: 7:

(i) CARACTERISTIQUES DE LA SEQUENCE:
   (A) LONGUEUR: 24 acides aminés
   (B) TYPE: acide aminé
   (D) CONFIGURATION: linéaire
(xi) DESCRIPTION DE LA SEQUENCE: SEQ ID NO: 7:
   Lys Tyr Arg Glu Lys Asn Ser Lys Asp Gly Lys Lys Lys Lys Lys Lys Ser
    1               5                  10                  15
   Lys Thr Lys Cys Ile Ile Met
           20

### (2) INFORMATION POUR LA SEQ ID NO: 8:

(i) CARACTERISTIQUES DE LA SEQUENCE:
   (A) LONGUEUR: 9 acides aminés
   (B) TYPE: acide aminé
   (D) CONFIGURATION: linéaire
(xi) DESCRIPTION DE LA SEQUENCE: SEQ ID NO: 8:
   Arg Lys Lys Arg Arg Gln Arg Arg Arg
             1               5

### (2) INFORMATION POUR LA SEQ ID NO: 9:

(i) CARACTERISTIQUES DE LA SEQUENCE:
   (A) LONGUEUR: 16 acides aminés
   (B) TYPE: acide aminé
   (D) CONFIGURATION: linéaire
(xi) DESCRIPTION DE LA SEQUENCE: SEQ ID NO: 9:
   Arg Gln Ala Arg Arg Asn Arg Arg Arg Arg Trp Arg Glu Arg Gln Arg
    1               5                  10                  15

### (2) INFORMATION POUR LA SEQ ID NO: 10:

(i) CARACTERISTIQUES DE LA SEQUENCE:
   (A) LONGUEUR: 6 acides aminés
   (B) TYPE: acide aminé
   (D) CONFIGURATION: linéaire
(xi) DESCRIPTION DE LA SEQUENCE: SEQ ID NO: 10:
   Arg Ala Pro Arg Lys Lys
    1               5

## Description

La présente invention concerne des séquences nucléiques, les vecteurs les contenant, et leurs utilisations thérapeutiques, notamment en thérapie génique. Plus particulièrement, la présente invention concerne des séquences nucléiques comprenant un gène codant pour une protéine intracellulaire de liaison (PIL), et leur utilisation en thérapie génique, éventuellement incorporées à des vecteurs d'expression appropriés.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. A cet égard, différentes techniques de transfection et de transfert de gènes ont été décrites dans la littérature (Cf Roemer et Friedman, Eur. J. Biochem. 208 (1992) 211). Jusqu'à aujourd'hui, les approches proposées dans l'art antérieur pour la thérapie génique consistent à transférer des gènes codant pour des polypeptides actifs impliqués dans des maladies génétiques (hormones, facteurs de croissance, etc), des gènes antisens, ou des peptides antigéniques pour la réalisation de vaccins. La présente invention concerne une nouvelle approche de la thérapie génique, consistant à transférer et à exprimer dans une cellule (ou un tissu) cible un peptide intracellulaire capable d'interagir avec des composants cellulaires et ainsi d'interférer avec des fonctions cellulaires. La présente invention repose plus particulièrement sur la mise en évidence qu'il est possible d'exprimer in vivo des anticorps modifiés qui restent dans le compartiment intracellulaire, et qui peuvent contrôler certaines fonctions cellulaires. L'invention repose également sur la mise en évidence qu'il est possible de cloner des séquences d'ADN codant pour de tels anticorps intracellulaires dans des vecteurs, notamment viraux, pour une utilisation en thérapie génique.

L'utilisation d'anticorps en thérapie humaine permet, en général, de cibler et neutraliser des complexes biologiques circulants et/ou localisés à la surface des cellules, en entrainant une cascade d'événements gérés par le système immunitaire qui conduit à leur élimination. Cependant, dans beaucoup de cas, dont les cancers ou les maladies dues par exemple à des virus, cette approche est stérile car l'antigène responsable de la dérégulation des cellules atteintes est inaccessible aux anticorps responsable de la dérégulation des cellules atteintes est inaccessible aux anticorps injectés. La présente invention offre une nouvelle approche thérapeutique particulièrement avantageuse, consistant à faire produire de manière continue et intracellulaire des anticorps ou agents thérapeutiques dont la liaison à leur épitope décroit et/ou annule la dérégulation.

La possibilité d'exprimer de manière recombinante des anticorps a déjà été décrite dans la littérature. Ainsi, le brevet EP 88994 décrit l'expression in vitro et la purification de régions variables de chaines lourdes ou légères d'anticorps. De même, le brevet US 4,946,778 décrit l'expression in vitro de séquences d'ADN codant pour des anticorps modifiés composés de régions variables de chaines lourde et légère d'un anticorps reliées par un linker. Cependant, les anticorps décrits dans ce brevet sont inactifs, et généralement synthétisés sous forme de corps d'inclusion insolubles. Les anticorps doivent donc être purifiés puis soumis à des traitements chimiques (dénaturation, renaturations, etc) pour recouvrer une activité. La présente invention démontre la possibilité d'utiliser de telles séquences d'ADN pour l'expression directement in vivo d'anticorps intracellulaires actifs. La présente invention démontre ainsi la possibilité d'utiliser de telles séquences d'ADN codant pour des anticorps intracellulaires, sous le controle de régions permettant leur expression dans les cellules mammifères, pour une thérapie génique, notamment chez l'homme. Cette nouvelle approche permet donc de cibler des composants cellulaires non accessibles par les méthodes classiques de vaccination. De plus, cette approche n'implique pas le développement d'une réponse immunitaire, mais agit intracellulairement.

Le document DE 3 915 952 mentionne la possibilité d'utiliser des anticorps intracellulaires introduits par leur séquence nucléotidique pour inhiber l'activité de protéines endogènes cependant ce document ne fournit aucune indication selon laquelle l'anticorps intracellulaire peut être un ScFv tel que décrit dans la présente invention.

Un premier objet de l'invention réside donc dans une séquence d'acides nucléiques comprenant un gène codant pour une protéine intracellulaire de liaison (PIL) sous le controle de régions permettant son expression dans les cellules mammifères en ce que:
a) la PIL est un peptide comprenant au moins un peptide correspondant au site de liaison de la région variable de la chaine légère d'un anticorps relié par un linker peptidique à un peptide correspondant au site de liaison de la région variable de la chaine lourde d'un anticorps
b) la PIL est un peptide dirigé contre un oncogène ou contre un facteur de la voie de signalisation d'un oncogène

L'invention concerne également des vecteurs contenant une séquence d'acides nucléiques telle que définie ci-dessus. Plus particulièrement, les vecteurs de l'invention sont d'origine virale, tels que les rétrovirus, les adénovirus, les virus adéno-associés, le virus de l'herpès, le virus de la vaccine, le virus HSV, etc.

L'invention concerne également l'utilisation de ces séquences d'acides nucléiques ou de ces vecteurs pour la préparation de compositions pharmaceutiques destinées au traitement chirurgical et/ou thérapeutique du corps humain ou animal. Elle concerne aussi toute composition pharmaceutique comprenant un vecteur, notamment viral, ou une séquence d'acides nucléiques tels que définis ci-dessus.

Au sens de la présente invention, le terme protéine intracellulaire de liaison (PIL) désigne toute protéine ou fragment de protéine capable de reconnaitre un composant de la cellule dans laquelle elle est exprimée, et d'interagir de manière sélective et affine avec celui-ci, Il peut s'agir d'interactions chimiques covalentes ou non covalentes. L'interaction avec le composant cellulaire (protéines, lipides, acides aminés, ARNm, ARNt, ARNr, ADN, etc) permet d'agir sur une fonction cellulaire dans laquelle est impliqué ledit composant, et ainsi de contrôler (stimuler, ralentir, inhiber) cette fonction.

Préférentiellement, les PIL selon l'invention sont constituées par des molécules dérivées d'anticorps ou ayant des propriétés de liaison comparables à celles d'un anticorps. En particulier, il s'agit de protéines ayant une sélectivité et une affinité suffisantes pour permettre une interaction in vivo ayant un effet neutralisant sur l'antigène. Ces molécules sont désignées dans ce qui suit anticorps intracellulaires, en raison de leurs propriétés et de leur localisation.

Les anticorps, molécules de la superfamille des immunoglobulines, sont synthétisés naturellement (essentiellement par les lymphocytes B) sous forme de protéines sécrétées. Ils sont donc libérés dans les compartiments extracellulaires (système circulatoire) où ils exercent leur activité (reconnaissance et liaison aux antigènes non-soi). Il a maintenant été montré qu'il est possible d'exprimer in vivo des gènes modifiés codant pour des anticorps intracellulaires, sans affecter les propriétés de spécificité et d'affinité des anticorps. Les séquences d'acides nucléiques selon l'invention, qui codent pour des anticorps intracellulaires, comprennent donc un gène d'anticorps modifié de façon à ce que l'anticorps ne soit pas sécrété. En particulier, le gène de l'anticorps est généralement modifié par délétion ou mutation des séquences responsables de sa sécrétion. Les PIL selon l'invention peuvent notamment être constituées de fragments d'anticorps, et par exemple de fragments Fab ou F(ab)'2 qui portent les domaines de liaison de l'antigène. L'utilisation de ce type d'anticorps intracellulaire implique cependant l'expression d'une séquence d'acides nucléiques comprenant plusieurs gènes codant respectivement pour les régions lourdes et légères de ces fragments, et elle implique également que ces chaires s'assemblent correctement in vivo. Pour cette raison, une forme particulièrement avantageuse d'anticorps intracellulaires utilisables dans le cadre de l'invention est constituée d'un peptide correspondant au site de liaison de la région variable de la chaîne légère d'un anticorps relié par un linker peptidique à un peptide correspondant au site de liaison de la région variable de la chaire lourde d'un anticorps. L'utilisation de ce type d'anticorps intracellulaire, désigné ScFv, est interessante car ils sont exprimés par un gène unique. La construction de séquences d'acides nucléiques codant pour de tels anticorps modifiés selon l'invention est illustrée dans les exemples.

Par ailleurs, les séquences d'acides nucléiques codant pour les anticorps intracellulaires selon l'invention peuvent également être modifiées par voie chimique, enzymatique ou génétique, en vue de gènèrer des anticorps intracellulaires stabilisés, et/ou multifonctionnels, et/ou de taille réduite, et/ou dans le but de favoriser leur localisation dans tel ou tel compartiment intracellulaire. Ainsi, les séquences d'acides nucléiques de l'invention peuvent comprendre des séquences codant pour des peptides de localisation nucléaire (NLS). En particulier, il est possible de fusionner les séquences de l'invention avec la séquence codant pour le NLS du virus SV40, dont la séquence peptidique est la suivante : MPKKKRK (Kalderon et al, Cell 39 (1984) 499).

Comme indiqué plus haut, les séquences d'acides nucléiques selon l'invention comprennent des séquences permettant l'expression du ou des gène(s) codant pour les PIL dans les cellules mammifères. Généralement, les gènes PIL sont donc placés sous le contrôle de régions promotrices de la transcription et de la traduction, fonctionnelles dans la cellule mammifère dans laquelle l'expression est recherchée. Il peut s'agir de séquences homologues vis-à-vis de ladite cellule, c'est à dire de séquences responsables naturellement de l'expression de gènes dans ladite cellule. Il peut également s'agir de séquences d'origine différente, c'est à dire de séquences responsables de l'expression de protéines dans d'autres types cellulaires, de séquences responsables de l'expression d'anticorps dans les conditions naturelles, de séquences d'expression virales, par exemple présentes dans un vecteur dans lequel les séquences de l'invention sont incorporées, ou encore de séquences synthétiques ou semi-synthétiques.

S'agissant d'un usage pour l'homme, de nombreux promoteurs fonctionnels ont été décrits dans la littérature, tels que par exemple les promoteurs viraux CMV, SV40, Ela, MLP, LTR, etc. Des promoteurs cellulaires, tels que par exemple le promoteur du gène de la villine, présente un intérêt car permettent une expression tissu spécifique (limitée à l'intestin dans le cas de la villine).

Par ailleurs, les séquences d'expression peuvent également être modifiées, par exemple pour les adapter à l'expression dans un type particulier de vecteur ou de cellule, pour réduire leur taille, pour augmenter leur activité de promoteur de la transciption, pour générer des promoteurs inductibles, améliorer leur niveau de régulation, ou encore changer la nature de leur régulation. De telles modifications peuvent être effectuées par exemple par mutagénèse *in vitro,* par introduction d'éléments additionnels de contrôle ou de séquences synthétiques, ou par des délétions ou des substitutions des éléments originels de contrôle. Il peut être particulièrement avantageux d'utiliser des promoteurs tissu-spécifiques, afin de cibles l'expression de la PIL dans un type seulement de tissu.

Par ailleurs, lorsque la séquence d'acides nucléiques ne comporte pas de séquences d'expression, celle-ci peut être inscorporée dans un vecteur d'expression, en aval d'une telle séquence.

Pour préparer un vecteur selon l'invention, il convient, dans un premier temps, d'identifier une fonction cellulaire, par exemple impliquée dans ou responsable d'une pathologie, sur laquelle on désire agir. Ensuite, il convient d'identifier un composant cellulaire approprié impliqué dans cette fonction, puis de déterminer quel PIL semble le plus adapté à ce composant (anticorps, dérivés, etc), en fonction de sa localisation, de son rôle, de sa nature, etc. Le PIL ayant été sélectionné, une séquence d'acides nucléiques correspondante peut être obtenue par les techniques de biologie moléculaire (synthèse chimique, clonage, modification enzymatique, etc), et insérée dans un vecteur appropriée selon la méthodologie décrite dans les exemples.

Un autre objet de l'invention concerne des compositions pharmaceutiques comprenant au moins une séquence d'acides nucléiques ou un vecteur tels que définis précédemment.

Les séquences de l'invention peuvent être utilisées telles quelles, par exemple après injection à l'homme ou l'animal, pour induire l'expression intracellulaire d'une PIL en vue d'affecter une fonction cellulaire déterminée. En particulier, elles peuvent être injectées sous forme d'ADN nu selon la technique décrite dans la demande WO 90/11092. Elles peuvent également être administrées sous forme complexée, par exemple avec du DEAE-dextran (Pagano et al., J.Virol. 1 (1967) 891), avec des protéines nucléaires (Kaneda et al., Science 243 (1989) 375), avec des lipides (Felgner et al., PNAS 84 (1987) 7413), sous forme de liposomes (Fraley et al., J.Biol.Chem. 255 (1980) 10431), etc.

Dans un mode de réalisation préféré de l'invention, les séquences nucléiques telles que définies précédemment sont incorporées dans un vecteur. L'emploi de tels vecteurs permet en effet de favoriser la pénétration dans les cellules, d'accroitre la résistance aux enzymes, et d'augmenter la stabilité et les niveaux d'expression intracellulaires. Les vecteurs de l'invention peuvent être aussi bien plasmidiques que viraux. Cependant, on préfère utiliser un vecteur viral.

Dans un mode de réalisation préféré, l'invention concerne donc des séquences d'acides nucléiques telles que définies précédemment incorporées à un vecteur viral. L'invention concerne également tout virus recombinant comprenant, inséré dans son génome, au moins une séquence d'acides nucléiques codant pour une PIL.

Comme indiqué plus haut, différents virus sont susceptibles d'être utilisés comme vecteurs pour le transfert et l'expression in vivo de gènes selon l'invention. A titre d'exemple, on peut citer les rétrovirus (RSV, HMS, MMS, etc), le virus HSV, les virus adéno-associés, les adénovirus, le virus de la vaccine etc.

Avantageusement, le virus recombinant selon l'invention est un virus défectif. Le terme "virus défectif" désigne un virus incapable de se répliquer dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par les séquences nucléiques de l'invention. Préférentiellement, le virus défectif conserve néanmoins les séquences de son génome qui sont nécessaires à l'encapsidation des particules virales.

Des virus recombinants défectifs dérivés de rétrovirus, de virus adéno-associés, du virus HSV (herpes simplex virus) ou des adénovirus ont déjà été décrits dans la littérature [Roemer et Friedmann, Eur. J. Biochem. 208 (1992) 211; Dobson et al., Neuron 5 (1990) 353; Chiocca et al., New Biol. 2 (1990) 739; Miyanohara et al., New Biol. 4 (1992) 238; WO91/18088; Akli et al., Nature Genetics 3 (1993) 224; Stratford-Perricaudet et al., Human Gene Therapy 1 (1990) 241; EP 185 573, Levrero et al., Gene 101 (1991) 195; EP 243204)].

Il est particulièrement avantageux d'utiliser les séquences nucléiques de l'invention sous forme incorporée à un adénovirus recombinant défectif.

Il existe en effet différents sérotypes d'adénovirus, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Par ailleurs, la faible taille des gènes codant pour les anticorps intracellulaires selon l'invention permet de manière avantageuse d'incorporer simultanément, dans un même vecteur, plusieurs gènes codant pour des anticorps intracellulaires dirigés contre des régions différentes d'un ou de plusieurs composants cellulaires ciblés. Un mode de réalisation particulier de l'invention consiste donc dans un vecteur, notamment viral, comprenant au moins deux séquences d'acides nucléiques codant pour des protéines intracellulaires de liaison dirigées contre des épitopes différents.

Les virus recombinants défectifs de l'invention peuvent être préparés par recombinaison homologue entre un virus défectif et un plasmide portant entre autre la séquence d'acides nucléiques telle que définie ci-avant (Levrero et al., Gene 101 (1991) 195; Graham, EMBO J. 3(12) (1984) 2917). La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus recombinants défectifs, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). A titre d'exemple de lignée utilisable pour la préparation de rétrovirus recombinants défectifs, on peut mentionner la lignée CRIP (Danos et Mulligan, PNAS 85 (1988) 6460).

Ensuite, les virus qui se sont multipliés sont récupérés et purifiés selon les techniques classiques de biologie moléculaire.

La présente invention concerne donc également une composition pharmaceutique comprenant au moins un virus recombinant défectif tel que défini précédemment.

Les compositions pharmaceutiques de l'invention peuvent être formulées en vue d'une administration par voie topique, orale, parentérale, intranasale, intraveineuse, intramusculaire, sous-cutanée, intraoculaire, etc.

Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses d'acides nucléiques (séquence ou vecteur) utilisées pour l'administration peuvent être adaptées en fonction de différents paramètres, et notamment en fonction du mode d'administration utilisé, de la pathologie concernée, du gène à exprimer, ou encore de la durée du traitement recherchée. D'une manière générale, concernant les virus recombinants selon l'invention, ceux-ci sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

L'invention a également pour objet toute cellule recombinante comprenant une séquence d'acides nucléiques telle que définie ci-avant.

Les séquences de l'invention, éventuellement incorporées à des vecteurs, et les compositions pharmaceutiques les contenant peuvent être utilisées pour le traitement de nombreuses pathologies. Elles peuvent donc être utilisées pour le transfert et l'expression de gènes in vivo dans tout type de tissu. Le traitement peut d'ailleurs être ciblé en fonction de la pathologie à traiter (le transfert au niveau d'un tissu particulier peut notamment être déterminé par le choix d'un vecteur, et l'expression par le choix d'un promoteur particulier). Les séquences ou vecteurs de l'invention sont avantageusement utilisées pour la production chez l'homme ou l'animal, in vivo et de manière intracellulaire, de protéines capables d'agir de manière spécifique sur des fonctions cellulaires diverses telles que la prolifération cellulaire, la synthèse de métabolites, la synthèse de protéines, la réplication et/ou la transcription de l'ADN, etc. La présente invention permet ainsi de traiter de manière spécifique, locale et efficace, de nombreux dysfonctionnements cellulaires à l'origine ou résultant de différentes pathologies, et en particulier les cancers, maladies virales ou bactériennes, ou, plus généralement, toute pathologie dans laquelle un médiateur cellulaire peut être identifié.

### Utilisation pour le traitement de pathologies liées à la prolifération cellulaire

Dans un mode particulièrement avantageux, les séquences d'acides nucléiques de l'invention comprennent des gènes codant pour des PIL capables d'interagir et d'interférer avec l'activité de facteurs impliqués dans la prolifération cellulaire. La prolifération cellulaire met en jeu une multitude de facteurs, tels que des récepteurs membranaires (protéines G), des oncogènes, des enzymes (protéines kinases, farnésyl transférases, phospholipases, etc) des nucléosides (ATP, AMP, GDP, GTP, etc) des facteurs d'activation [facteurs d'échange des guanosines (GRF, GAP, etc), facteurs transcriptionnels, etc], etc. L'expression intracellulaire de PIL selon l'invention capables de lier et de neutraliser de tels facteurs permet de controler le processus de prolifération cellulaire. Ceci est particulièrement intéressant dans les situations où la prolifération cellulaire échappe aux mécanismes naturels de régulation, conduisant par exemple à l'apparition de tumeurs. De nombreux facteurs (produits des gènes oncogènes et facteurs impliqués dans la signalisation de l'effet des ces produits) ont en effet été associés à ces phénomènes de dérégulation de la prolifération cellulaire. Ainsi, 90 % des adénocarcinomes du pancréas présentent un oncogène Ki-ras muté sur le douzième codon (Almoguera et coll., Cell 53 (1988) 549). De même, la présence d'un gène ras muté a été mise en évidence dans les adénocarcinomes du colon et les cancers de la thyroïde (50 %), ou dans les carcinomes du poumon et les leucémies myéloïdes (30 %, Bos, J.L. Cancer Res. 49 (1989) 4682). De nombreux autres oncogènes ont aujourd'hui été identifiés (myc, fos, jun, ras, myb, erb, etc), dont des formes mutées semblent responsables d'un dérèglement de la prolifération cellulaire.

L'expression de PIL capables de lier ces facteurs cellulaires (préférentiellement leur forme oncogénique) et donc de ralentir ou d'inhiber leurs effets offre la possibilité d'une nouvelle approche thérapeutique des cancers.

Dans un mode particulièrement intéressant, la présente invention concerne des vecteurs contenant des séquences d'acides nucléiques comprenant un gène codant pour un anticorps intracellulaire capable d'interagir avec le produit d'expression d'un oncogène ou avec un facteur intervenant dans la voie de signalisation d'un oncogène.

Parmi les oncogènes cibles, on peut citer au sens de l'invention les oncogènes ras, fos, jun, myc, myb et erb.

Parmi les facteurs intervenant dans la voie de signalisation d'un oncogène, on peut citer notamment les récepteurs membranaires, qui peuvent être ciblés au niveau de leurs domaines intracellulaires [protéines G, kinases (exemple : tyrosine kinase), phosphorylases, farnésyl transférases], les facteurs d'échange des nucléosides (facteurs GAP, GRF, etc), etc.

Plus préférentiellement, la présente invention concerne des vecteurs, notamment d'origine virale, contenant une séquence d'acides nucléiques codant pour un anticorps intracellulaire dirigé contre un oncogène ou un facteur intervenant dans la voie de signalisation d'un oncogène, constitué d'un peptide correspondant au site de liaison de la région variable de la chaine légère d'un anticorps relié par un linker peptidique à un peptide correspondant au site de liaison de la région variable de la chaine lourde d'un anticorps.

Plus particulièrement, l'invention concerne des virus recombinants défectifs exprimant un anticorps intracellulaire dirigé contre un facteur de la voie de signalisation ras-dépendante.

Comme le montrent les exemples, l'expression d'anticorps intracellulaires anti-p21 (produit d'expression du gène ras), anti-GAP, ou anti-p53 permet de réverter le phénotype transformant d'une cellule cancéreuse.

En outre, comme indiqué plus haut, la faible taille des gènes codant pour l'anticorps intracellulaire selon l'invention permet de manière avantageuse d'exprimer simultanément, dans un même vecteur plusieurs anticorps intracellulaires dirigés contre des régions différentes d'une ou de plusieurs de ces cibles.

Les séquences nucléiques selon l'invention peuvent également être des séquences codant pour des PIL capables d'interagir et d'interférer avec l'activité de facteurs impliqués dans la synthèse de métabolites, dans la synthèse protéique ou encore dans la réplication et/ou transcription de l'ADN.

La présente invention sera plus complètement décrite à l'aide des exemples qui suivent, qui doivent être considérés comme illustratifs et non limitatifs.

### Légende des figures

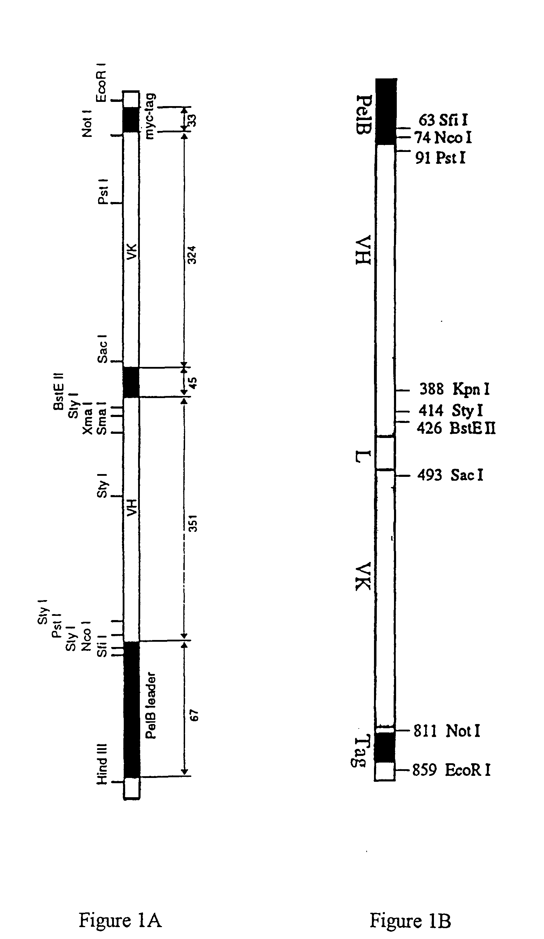

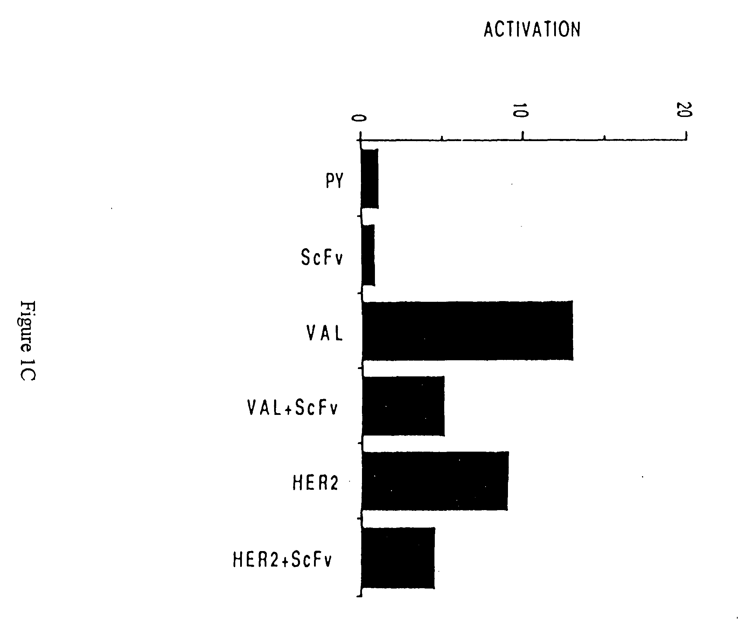

Figure 1 : (A) Carte de restriction du ScFv-ras. (B) Carte de restriction du ScFv-Gap. (C) Effet neutralisant de l'expression transitoire d'un anticorps intracellulaire de l'invention sur le pouvoir transformant d'un oncogène ras ou Her2. Py = cellules controle; ScFv = cellules transectées par le plasmide psv2.ScFv.ras seul, VAL = cellules transectées par le vecteur exprimant ras oncogénique Ha-ras Val12; VAL+ScFv = cellules cotransectées par le plasmide psv2.ScFv.ras et Ha-ras Val12; HER2 = cellules transectées par le vecteur exprimant Her2 oncogénique; HER2+ScFv = cellules cotransectées par le plasmide psv2.ScFv.ras et par Her2.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérsse-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemple 1 : Clonage et expression d'une séquence d'ADN codant pour un anticorps intracellulaire anti-ras

Cet exemple décrit le clonage et l'expression d'une séquence d'acides nucléiques codant pour protéine intracellulaire de laison reproduisant les propriétés de l'anticorps monoclonal Y13-259. L'anticorps Y13-259 est dirigé contre les protéines Ras (ref. ATCC CRL 1742) (J. Virol. 43, 294-304, 1982), et est un anticorps neutralisant sur la fonction des protéines Ras oncogéniques une fois injecté dans les cellules [Smith et coll., (1986) Nature, 320, 540-543 ; Kung et coll., Exp. Cell. Res. (1986) 162, 363-371].

### 1.1. Préparation de la séquence d'ADN

Une séquence d'ADN codant pour un anticorps intracellulaire (fragment ScFv) a été préparée selon la technique décrite dans le brevet US 4,946,778. Cette séquence a ensuite été placée sous le controle d'un promoteur fonctionnel dans les cellules mammifère.

Les ARN poly A sont isolés à partir d'une culture cellulaire de l'hybridome qui secrète l'anticorps Y13-259 selon la technique décrite par Chirguin S.H. et al. [Biochemistry 18, 5294 (1979)]. Ces ARN sont utilisés pour une réaction de Reverse-Transcription à l'aide de primers formés d'hexanucléotides aléatoires. Les ADNc obtenus servent de matrice à deux réactions de PCR :
- l'une destinée à amplifier le fragment variable de la chaîne lourde (VH) de Y13-259 avec des primers spécifiques de VH murins,
- la seconde permettant d'obtenir le fragment VL en utilisant un mélange de 10 primers dérivés des séquences murines.

Deux fragments de 340 pb et 325 pb sont ainsi obtenus et ensuite assemblés grâce à un linker qui permet un positionnement correct du cDNA de VH en 5' de celui de VL. Ce linker code pour 15 acides aminés formés de trois répétitions du motif (Gly)₄ Ser [Orlandi, R. et al., Proc. Natl. Acad. Sci. USA, 86, 3833-3837 (1979)]. La séquence de l'anticorps intracellulaire est présentée sur la SEQ ID n° 1 (résidus 28 à 270). Cette séquence confère assez de degrés de liberté à la fusion VH-VL pour permettre leur assemblage en orientation parallèle et assurer une affinité correcte pour l'antigène.

La séquence d'acides nucléiques fusionnée VH-linker-VL est ensuite insérée dans un phagemide qui permet l'expression de l'anticorps intracellulaire (fragment ScFv) à la surface d'un phage M13 (figure 1A). Cette expression permet facilement l'identification et la sélection des anticorps intracellulaires qui reconnaissent correctement l'antigène.

### 1.2. Evaluation fonctionnelle de l'anticorps intracellulaire modifié.

La séquence d'ADN qui code pour l'anticorps intracellulaire modifié anti-Ras (VH-linker-VL) est isolée du phagemide par restriction, puis insérée dans le vecteur sv2 sous contrôle du couple enhancer promoteur précoce de SV40 (Schweighoffer et al. Science, 256, 825-827, 1992), afin de tester sa capacité à antagoniser les effets d'un Ras oncogénique. Le plasmide ainsi obtenu est désigné psv2.ScFv.ras. L'évaluation fonctionnelle a été effectuée selon plusieurs tests :

### a) Transfection transitoire dans les cellules mammifères.

Pour l'évaluation par transfection transitoire dans les cellules mammifères, le plasmide psv2.ScFv.ras a été cotransfecté dans les cellules NIH 3T3 selon le protocole décrit dans Schweighoffer et al. [Science, 256, 825-827 (1992)] avec un vecteur qui permet l'expression d'un gène Ha-ras Val 12. L'état d'activation de la voie de signalisation étudiée a été enregistré par mesure de l'activité enzymatique obtenue à partir du gène rapporteur "chloramphenicol-acetyl transferase (CAT)" placé sous le contrôle d'un promoteur contenant éléments nucléotidiques répondant en "trans" à l'action de Ras (RRE) également cotransfectés : ces éléments RRE sont constitués par un promoteur hybride TK-enhancer du polyome (Wasylyk et coll., EMBO, J. 7, 2475, 1988).

Les résultats obtenus sont présentés sur la figure 1C. L'analyse des activités CAT obtenue démontre la capacité de l'anticorps intracellulaire modifié, préparé à partir de l'anticorps Y13-259, d'antagoniser l'activité du Ras oncogénique.

Le plasmide psv2.ScFv.ras cotransfecté avec un plasmide permettant l'expression d'un oncogène doué d'activité tyrosine kinase, l'oncogène HER2 (human epidermal growth factor type II) bloque également son activité sur le plasmide "CAT" test (figure 1).

### b) Formation de foyers de cellules transformées : Les cellules cancéreuses ont la propriété de former des foyers de transformation et notamment les fibroblastes NIH 3T3 exprimant un Ras oncogénique (Barlat et coll., Oncogene (1993), B, 215-218).

Les cellules NIH 3T3 sont cultivées comme dans le test précédent dans un milieu Dulbecco modifié Eagle (DMEM) contenant 10 % de sérum de veau fétal, à 37°C dans un environnement humide contenant 5 % de CO₂. Ces cellules sont ensuite co-transfectées avec un Ras oncogénique : Ha-ras Val12, le plasmide psv2.ScFv.ras (Cf a) ci-dessus), et un excès de 10 fois du gène de résistance à la néomycine, par la technique de transfection aux lipides cationiques (Schweighoffer et coll. Science, 256, 825-827, 1992). La même quantité totale d'ADN est transfectée pour chaque boîte.

24 heures après la transfection, les cellules transfectées provenant de chaque boîte de pétri de 100 mm sont divisées dans un rapport 1 à 10 et cultivées dans le même milieu mais en présence de G418 (GIBCO/BRL) à 0,4 mg par ml de milieu. Le nombre de foyers de transformation obtenu par µg d'ADN transfecté est comptabilisé après 14 jours de culture.

Les résultats obtenus sont présentés dans le tableau ci-dessous. Ils représentent la moyenne de quatre essais indépendants.

**TABLEAU**

| Transformation de cellules NIH 3T3 par Ha-ras Val12 en présence d'anticorps intracellulaire anti-ras | |
|---|---|
| Plasmides transfectés | Nombre de foyers par µg d'ADN transfecté |
| Ha-Ras Val12 | 110 |
| psv2.ScFv.ras | 2 |
| Ha-Ras Val12 + psv2.ScFv.ras | 30 |

Les résultats obtenus montrent clairement que l'anticorps intracellulaire anti-ras diminue très fortement le pouvoir transformant d'un gène ras oncogénique.

Par ailleurs, vu les résultats obtenus en a), l'expression de ce fragment ScFv de l'anticorps Y13-159 devrait également empêcher la transformation par d'autres oncogènes tel HER1, HER2 facilitant l'activation des protéines Ras cellulaires.

Il est entendu que l'homme du métier peut, sur la base des résultats décrits dans la présente demande, reproduire l'invention avec des séquences d'acides nucléiques codant pour des anticorps intracellulaires (tels que des fragments ScFv) dirigés contre d'autres composants cellulaires. Ceux-ci peuvent être préparés soit à partir d'anticorps connus dirigés contre des composants cellulaires, soit par identification d'un antigène à neutraliser, immunisation au moyen de cet antigène ou d'un épitope préféré de celui-ci, puis préparation de l'anticorps intracellulaire à partir de l'anticorps, de son ARNm, ou de hybridome obtenus. D'autres composants impliqués dans des processus de transformation cellulaire peuvent ainsi être ciblés. Par exemple, d'autres séquences d'ADN codant pour des anticorps intracellulaires de liason anti-ras peuvent être préparées selon la même méthodologie, à partir des hybridomes M38, M8, M70, M90 et M30 (ATCC HB 9158), dont les anticorps sont dirigés respectivement contre les résidus 1 à 23, 24 à 69, 90 à 106, 107 à 130 et 131 à 152 de la protéine Ha-Ras. De plus, comme indiqué plus haut, des vecteurs portant simultanément plusieurs séquences codant pour différents anticorps intracellulaires peuvent être avantageusement préparés, pour conférer une activité neutralisante supérieure.

### Exemple 2 : Clonage et expression d'une séquence d'ADN codant pour un anticorps intracellulaire anti-GAP

Cet exemple décrit le clonage et l'expression d'une séquence d'acides nucléiques codant pour une protéine intracellulaire de laison reproduisant les propriétés d'un anticorps monoclonal dirigé contre la protéine GAP.

La protéine GAP (pour GTPase Activating Protein) est impliquée dans la voie de signalisation ras-dépendante. Elle interagit avec les protéines ras de manière catalytique et multiplie par 100 à 200 la vitesse d'hydrolyse du GTP mesurée *in vitro* pour la protéine p21 normale. Différents travaux ont montré que le domaine catalytique de cette protéine de 1044 acides aminés environ était situé dans la région carboxy-terminale (résidus 702-1044), et que cette région était responsable de l'interaction de la protéine GAP avec les protéines ras (Cf WO91/02749).

Il a maintenant été montré qu'un anticorps monoclonal dirigé contre le domaine dit "SH3" de la protéine GAP neutralisait les fonctions des protéines Ras oncogéniques dans l'oeuf de Xénope (Duchesne et coll., Science, 259, 525-528, 1993).

Selon la méthodologie décrite en 1.1., il est possible de synthétiser une séquence d'ADN codant pour un anticorps intracellulaire (fragment ScFv) correspondant à cet anticorps (SEQ ID n° 2, résidus 11 à 250, figure 1B). Une telle séquence, incorporée à un vecteur peut permettre d'inhiber le pouvoir transformant d'un gène ras oncogénique dans les cellules tumorales.

Par ailleurs, la demanderesse a également identifié plus précisément les épitopes reconnus par cet anticorps. Ces épitopes ont ensuite été synthétisés artificiellement, et peuvent être utilisés pour générer de nouveaux anticorps neutralisants pouvant servir à la mise en oeuvre de l'invention.

### a) Identification plus précise :

L'identification a été réalisée par la technique "d'epitope scanning". Cette technique est basée sur le principe qu'un anticorps donné peut réagir avec des peptides de 5 à 15 acides aminés. De ce fait, l'identification d'épitopes séquentiels peut être obtenue en préparant un jeu complet de peptides recouvrants, de 5-15 acides aminés, correspondants à la séquence complète de l'antigène considéré. Cette technique a été utilisée pour déterminer les épitopes fonctionnels du domaine "SH3" de GAP. Pour cela, la totalité de ce domaine a été explorée par recouvrements séquentiels, par synthèse d'un décapeptide tous les 2 acides aminés.
- Synthèse des peptides recouvrants.
   35 peptides couvrant la totalité du fragment de la figure 1 ont été synthétisés chimiquement. La synthèse a été effectuée en double, sur 2 supports indépendants, par la méthode Fmoc/t-butyl sur phase solide (kit Cambridge Research Biochemicals).
- Mise en évidence des épitopes fonctionnels.
   Les épitopes fonctionnels reconnus par l'anticorps Ac200 ont été révélés en test ELISA par un anticorps de lapin anti-souris couplé à la peroxydase. Le substrat chromogénique de l'enzyme utilisé est l'amino-di-(3-ethylbenzothiazodine sulfonate) (ABTS).

Les résultats obtenus montrent que les épitopes reconnus par cet anticorps sont les suivants :
- PVEDRRRVRAI (SEQ ID n° 3)
- EISF (SEQ ID n° 4)
- EDGWM (SEQ ID n° 5)

Ces épitopes peuvent être utilisés selon les techniques classiques de l'homme du métier pour générer des anticorps neutralisant les effets de ras. Ces anticorps ou hybridomes les produisant sont ensuite utilisés pour générer des séquences d'acides nucléiques et des vecteurs de l'invention, selon la méthodologie décrite précédemment.

### Exemple 3 : Préparation d'une séquence d'acides nucléiques codant pour un anticorps intracellulaire anti-Ki-ras à partir d'ARNm extraits de rates de souris immunisées avec des peptides dérivés des parties hypervariables de Ki-Ras (2A et 2B)

Cet exemple décrit la, préparation de séquences d'acides nucléiques codant pour des anticorps intracellulaires (tels que des fragments ScFv) selon l'invention par identification d'un antigène à neutraliser, immunisation au moyen de cet antigène ou d'un épitope préféré de celui-ci, puis préparation de la séquence d'acides nucléiques à partir de l'anticorps, de son ARNm, ou de hybridome obtenus.

Cet exemple démontre la possibilité d'appliquer la présente invention à tout antigène ou épitope désiré, même lorsqu'aucun anticorps monoclonal dirigé contre ledit antigène ou épitope n'est disponible.

L'antigène ciblé dans cet exemple est la protéine Ki-ras. Plus précisément, les antigènes utilisés pour l'immunisation sont les peptides de 25 et 24 acides aminés correspondant aux extrémités terminales des protéines Ki-Ras 2A et 2B suivants :

Après immunisation de souris avec ces peptides, selon les techniques classiques de l'immunologie, les rates sont extraites et les ADNc sont préparés à partir des ARNm. Les ADNc codant pour les régions variables sont ensuite clonés, ce qui aboutit à la constitution d'une banque de phages exprimant les ScFv correspondant à la totalité du répertoire des souris utilisées. Les anticorps intracellulaires (fragments ScFv) reconnaissant les peptides 2A et 2B sont ensuite identifiés et isolés par des étapes successives de sélection par affinité sur colonne et sur plaque de microtitration

Ces ScFv sont ensuite testés fonctionnellement selon le protocole décrit dans l'exemple 1.

La stratégie développée dans cet exemple permet d'une manière avantageuse de sélectionner des anticorps intracellulaires spécifiques des oncogènes Ki-Ras, qui n'affecteront donc pas les autres protooncogènes Ras. La sélectivité de tels outils est donc non seulement cellulaire (du fait du découplage des voies de transduction dans les cellules transformées par Ras) mais aussi moléculaire.

### Exemple 4 : Préparation d'une séquence d'acides nucléiques codant pour un anticorps intracellulaire anti-p53 mutées

Cet exemple décrit la préparation de séquences d'acides nucléiques codant pour des anticorps intracellulaires (tels que des fragments ScFv) dirigés contre des protéines p53 mutées. Ces anticorps intracellulaires sont obtenus à partir de différents anticorps monoclonaux dirigés contre lesdites protéines p53 mutées.

Le gène codant pour la protéine p53 est altéré dans un très grand nombre de cellules tumorales (Caron de Fromentel et Soussi, Genes, 4, 1-15, 1992). La protéine p53 mutée n'a pas la même conformation que la p53 sauvage (Lane et Benchimol, Genes Dev. 4, 1-8, 1990). Ce changement de conformation peut être détecté par des anticorps monoclonaux (Milner et Cook, Virology, 154, 21-30, 1986 ; Milner, Nature, 310, 143-145, 1984).

Les anticorps pAB 240 reconnaissent les formes mutées des protéines p53.

L'expression intracellulaire de fragment ScFv d'anticorps spécifiques de p53 mutée ou de protéines interagissant spécifiquement avec ces protéines p53 mutées devrait induire un effet bénéfique dans les tumeurs présentant une p53 mutée.

## Revendications

1. Séquence d'acides nucléiques comprenant un gène codant pour une protéine intracellulaire de liaison (PIL) sous le controle d'un promoteur fonctionnel dans les cellules mammifère **caractérisée en ce que** :
a) la PIL est un peptide comprenant au moins un peptide correspondant au site de liaison de la région variable de la chaine légère d'un anticorps relié par un linker peptidique à un peptide correspondant au site de liaison de la région variable de la chaine lourde d'un anticorps
b) la PIL est un peptide dirigé contre un oncogène ou contre un facteur de la voie de signalisation d'un oncogène

2. Séquence d'acides nucléiques selon la revendication 1 **caractérisée en ce qu'**elle code pour un peptide dirigé contre le produit d'expression d'un oncogène ras.

3. Séquence d'acides nucléiques comprenant tout ou partie de la séquence SEQ ID n° 1.

4. Séquence d'acides nucléiques selon la revendication 1 **caractérisée en ce qu'**elle code pour un peptide dirigé contre la protéine GAP.

5. Séquence d'acides nucléiques comprenant tout ou partie de la séquence SEQ ID n° 2.

6. Séquence d'acides nucléiques selon la revendication 1 **caractérisée en ce qu'**elle code pour un peptide dirigé contre une protéine p53.

7. Séquence d'acides nucléiques selon la revendication 1 **caractérisée en ce qu'**elle code pour un peptide dirigé contre une protéine p53 mutante.

8. Séquence d'acides nucléiques selon l'une des revendications 1 à 7 **caractérisée en ce que** le promoteur fonctionnel dans les cellules mammifère est choisi parmi les promoteurs viraux, cellulaires ou artificiels.

9. Séquence d'acides nucléiques selon l'une des revendications 1 à 7 **caractérisée en ce qu'**elle est incorporée dans un vecteur.

10. Séquence d'acides nucléiques selon la revendication 9 **caractérisée en ce qu'**elle est incorporée dans un vecteur viral.

11. Virus recombinant défectif comprenant dans son génome une séquence d'acides nucléiques selon l'une des revendications 1 à 8.

12. Virus recombinant défectif selon la revendication 11 **caractérisé en ce qu'**il est choisi parmi les rétrovirus, les adénovirus, les virus adéno-associés, le virus de la vaccine et le virus HSV.

13. Virus recombinant défectif **caractérisé en ce qu'**il comporte dans son génome une séquence d'acides nucléiques comprenant un gène au moins codant pour un anticorps intracellulaire dirigé contre un oncogène ou contre un facteur de la voie de signalisation d'un oncogène et comprenant au moins un peptide correspondant au site de liaison de la région variable de la chaine légère d'un anticorps relié par un linker peptidique à un peptide correspondant au site de liaison de la région variable de la chaine lourde d'un anticorps.

14. Virus recombinant défectif selon la revendication 13 **caractérisé en ce que** l'oncogène est choisi parmi les oncogènes myc, myb, ras, fos, jun et erb.

15. Virus recombinant défectif selon les revendications 13 ou 14 **caractérisé en ce qu'**il est choisi parmi les rétrovirus, les adénovirus, les virus adéno-associés, le virus de la vaccine et le virus HSV.

16. Vecteur, notamment viral, comprenant au moins deux séquences d'acides nucléiques selon la revendication 1 codant pour des protéines intracellulaires de liaison dirigées contre des épitopes différents d'un ou de plusieurs antigènes.

17. Composition pharmaceutique comprenant au moins une séquence d'acides nucléiques selon l'une des revendications 1 à 8 ou un virus recombinant selon l'une des revendications 11 à 16.

18. Composition pharmaceutique selon la revendication 17 **caractérisée en ce qu'**elle comprend au moins une séquence d'acides nucléiques selon l'une des revendications 1 à 7 sous forme de liposome, de complexe avec des protéines nucléaires, des lipides ou du dextran, ou sous forme brute.

19. Utilisation d'une séquence d'acides nucléiques selon la revendication 1 pour la préparation d'une composition pharmaceutique destinée au traitement des cancers.

## Patentansprüche

1. Nukleinsäuresequenz, umfassend ein Gen, das ein intracelluläres Bindungsprotein (PIL) codiert, unter der Kontrolle eines in Säugerzellen funktionellen Promotors, **dadurch gekennzeichnet, dass:**
a) das PIL ein Peptid ist, umfassend mindestens ein Peptid entsprechend der Bindungsstelle der variablen Region der leichten Kette eines Antikörpers in weiterer Bindung durch einen Peptidlinker an ein Peptid entsprechend der Bindungsstelle der variablen Region der schweren Kette eines Antikörpers;
b) das PIL ein Peptid ist, das gegen ein Oncogen oder einen Faktor des Signalwegs eines Oncogens gerichtet ist.

2. Nukleinsäuresequenz nach Anspruch 1, **dadurch gekennzeichnet, dass** sie ein Peptid codiert, das gegen das Expressionsprodukt eines ras-Oncogens gerichtet ist.

3. Nukleinsäuresequenz, umfassend die Gesamtheit oder einen Teil der Sequenz SEQ ID NO:1.

4. Nukleinsäuresequenz nach Anspruch 1, **dadurch gekennzeichnet, dass** sie ein gegen das GAP-Protein gerichtetes Peptid codiert.

5. Nukleinsäuresequenz, umfassend die Gesamtheit oder einen Teil der Sequenz SEQ ID NO:2.

6. Nukleinsäuresequenz nach Anspruch 1, **dadurch gekennzeichnet, dass** sie ein gegen das Protein p53 gerichtetes Peptid codiert.

7. Nukleinsäuresequenz nach Anspruch 1, **dadurch gekennzeichnet, dass** sie ein gegen eine Mutante des Proteins p53 gerichtetes Peptid codiert.

8. Nukleinsäuresequenz nach einem der Ansprüche 1 bis 7, **dadurch gekennzeichnet, dass** der in den Säugerzellen funktionelle Promotor aus viralen, cellulären oder künstlichen Promotoren ausgewählt ist.

9. Nukleinsäuresequenz nach einem der Ansprüche 1 bis 7, **dadurch gekennzeichnet, dass** sie in einen Vektor eingebaut ist.

10. Nukleinsäuresequenz nach Anspruch 9, **dadurch gekennzeichnet, dass** sie in einen viralen Vektor eingebaut ist.

11. Rekombinantes defektes Virus, umfassend in seinem Genom eine Nukleinsäuresequenz nach einem der Ansprüche 1 bis 8.

12. Rekombinantes defektes Virus nach Anspruch 11, **dadurch gekennzeichnet, dass** es aus Retroviren, Adenoviren, Adeno-assoziierten Viren, Vacciniaviren und dem HSV-Virus ausgewählt ist.

13. Rekombinantes defektes Virus, **dadurch gekennzeichnet, dass** es in seinem Genom eine Nukleinsäuresequenz umfasst, die mindestens ein Gen umfasst, das einen intracellulären Antikörper codiert, der gegen ein Oncogen oder einen Faktor des Signalwegs eines Oncogens gerichtet ist, und mindestens ein Peptid umfasst, das der Bindungsstelle der variablen Region der leichten Kette eines Antikörpers entspricht, das durch einen Peptidlinker an ein Peptid gebunden ist, das der Bindungsstelle der variablen Region der schweren Kette eines Antikörpers entspricht.

14. Rekombinantes defektes Virus nach Anspruch 13, **dadurch gekennzeichnet, dass** das Oncogen aus den Oncogenen myc, myb, ras, fos, jun und erb ausgewählt ist.

15. Rekombinantes defektes Virus nach Anspruch 13 oder 14, **dadurch gekennzeichnet, dass** es aus Retroviren, Adenoviren, Adeno-assoziierten Viren, Vacciniaviren und dem HSV-Virus ausgewählt ist.

16. Vektor, insbesondere viral, umfassend mindestens zwei Nukleinsäuresequenzen nach Anspruch 1, die intracelluläre Bindungsproteine codieren, die gegen verschiedene Epitope eines oder mehrerer Antigene gerichtet sind.

17. Pharmazeutische Zusammensetzung, umfassend mindestens eine Nukleinsäuresequenz nach einem der Ansprüche 1 bis 8 oder ein rekombinantes Virus nach einem der Ansprüche 11 bis 16.

18. Pharmazeutische Zusammensetzung nach Anspruch 17, **dadurch gekennzeichnet, dass** sie mindestens eine Nukleinsäuresequenz nach einem der Ansprüche 1 bis 7 in Form von Liposomen, Komplexen mit Kernproteinen, Lipiden oder Dextran oder in roher Form umfasst.

19. Verwendung einer Nukleinsäuresequenz nach Anspruch 1 zur Herstellung einer pharmazeutischen Zusammensetzung zur Behandlung von Krebs.

## Claims

1. Nucleic acid sequence comprising a gene coding for an intracellular binding protein (IBP) under the control of a promoter which is functional in mammalian cells, **characterized in that**:
a) the IBP is a peptide comprising at least one peptide corresponding to the binding site of the light-chain variable region of an antibody linked via a peptide linker to a peptide corresponding to the binding site of the heavy-chain variable region of an antibody
b) the IBP is a peptide directed against an oncogene or against a factor in the pathway of signalling of an oncogene

2. Nucleic acid sequence according to Claim 1, **characterized in that** it codes for a peptide directed against the product of expression of a ras oncogene.

3. Nucleic acid sequence comprising all or part of the sequence SEQ ID No. 1.

4. Nucleic acid sequence according to Claim 1, **characterized in that** it codes for a peptide directed against the GAP protein.

5. Nucleic acid sequence comprising all or part of the sequence SEQ ID No. 2.

6. Nucleic acid sequence according to Claim 1, **characterized in that** it codes for a peptide directed against a p53 protein.

7. Nucleic acid sequence according to Claim 1, **characterized in that** it codes for a peptide directed against a mutant p53 protein.

8. Nucleic acid sequence according to one of Claims 1 to 7, **characterized in that** the promoter which is functional in mammalian cells is chosen from viral, cellular or artificial promoters.

9. Nucleic acid sequence according to one of Claims 1 to 7, **characterized in that** it is incorporated in a vector.

10. Nucleic acid sequence according to Claim 9, **characterized in that** it is incorporated in a viral vector.

11. Defective recombinant virus comprising in its genome a nucleic acid sequence according to one of Claims 1 to 8.

12. Defective recombinant virus according to Claim 11, **characterized in that** it is chosen from retroviruses, adenoviruses, adeno-associated viruses, vaccinia virus and HSV virus.

13. Defective recombinant virus, **characterized in that** it contains in its genome a nucleic acid sequence comprising a gene at least coding for an intracellular antibody directed against an oncogene or against a factor in the pathway of signalling of an oncogene and comprising at least one peptide corresponding to the binding site of the light-chain variable region of an antibody linked via a peptide linker to a peptide corresponding to the binding site of the heavy-chain variable region of an antibody.

14. Defective recombinant virus according to Claim 13, **characterized in that** the oncogene is chosen from the myc, myb, ras, fos, jun and erb oncogenes.

15. Defective recombinant virus according to Claims 13 or 14, **characterized in that** it is chosen from retroviruses, adenoviruses, adeno-associated viruses, vaccinia virus and HSV virus.

16. Vector, in particular a viral vector, comprising at least two nucleic acid sequences according to Claim 1, coding for intracellular binding proteins directed against different epitopes of one or more antigens.

17. Pharmaceutical composition comprising at least one nucleic acid sequence according to one of Claims 1 to 8 or one recombinant virus according to one of Claims 11 to 16.

18. Pharmaceutical composition according to Claim 17, **characterized in that** it comprises at least one nucleic acid sequence according to one of Claims 1 to 7, in the form of liposomes or of a complex with nuclear proteins, lipids or dextran, or in untreated form.

19. Use of a nucleic acid sequence according to Claim 1, for the preparation of a pharmaceutical composition intended for the treatment of cancers.
